# EUROPEAN PATENT APPLICATION

(11) **EP 2 367 000 A1**
(43) Date of publication of application: **21.09.2011**
(21) Application number: 10075100.7
(22) Date of filing: 04.03.2010
(51) Int. Cl.: G01N 33/50

(54) **High throughput analysis of T-cell receptor repertoires**

(71) Applicant: Charité Universitätsmedizin Berlin, 10117 Berlin (DE)
(72) Inventor: Volk, Hans-Dieter, 10117 Berlin (DE); Reinke, Petra, 10117 Berlin (DE); Babel, Nina, 13469 Berlin (DE)
(74) Representative: Schulz Junghans

(57) **Abstract**

A method for analysis of a patient's T cell repertoire is provided, whereby nucleic acid molecules encoding T cell receptor protein sequences are obtained from lymphocytes isolated from a patient, a plurality of said nucleic acid molecules obtained is sequenced, rendering sample TCR sequences, the sample TCR sequences are aligned with a TCR sequence data base, and sample TCR sequences positively aligning to data base sequences are selected as selected sample TCR sequences, and the frequency distribution of at least some of the selected sample TCR sequences is determined. A method is preferred whereby the TCR sequence data base is obtained by stimulating lymphocytes isolated from a patient in vitro with one or several antigens, selecting T cells responding to stimulation from said lymphocytes and obtaining a plurality of nucleic acid sequences encoding T cell receptor proteins from at least some of the T cells selected.

## Description

Quantitative analysis of antigen-specific T cells in peripheral blood and tissue promises new insights into the aetiology and pathogenesis of diseases. Monitoring and manipulation of T cell receptor distribution among antigen-specific T cells may also allow for novel approaches in settings ranging from transplant management to cancer therapy.

Current techniques for T-cell monitoring include ELISPOT, cytokine flow cytometry, and MHC class I and II tetramer analysis. These methods however suffer from drawbacks with regard to their practical utility in the clinical setting. Monitoring of T cells in peripheral blood by standard methods requires fresh blood samples that have to be processed immediately. In addition, methods restricted to cells obtained from blood preclude analysis of tissue infiltrates, an important source of immune status information for transplant monitoring and other applications. Obtaining quantitative results requires laborious procedures and expensive reagents and machinery, and is confined to addressing problems where immunodominant epitopes and their HLA restriction are known and reagents exist for their analysis.

A method for the analysis of clonotypic composition in cytomegalovirus (CMV) specific CTL by manual sequencing of the variable Vβ chain CDR3 region of T cell receptors (TCR) is shown in Babel et al (J. Am. Soc. Nephrol. 20; 344-352, 2009). Therein, mono-or oligoclonal restriction of the TCR repertoire in CMV-specific T cells is analyzed by subcloning and traditional dideoxy sequencing after stimulation with a whole CMV pp 65 peptide library. The methods used in this work, however, are not satisfactory for clinical T-cell response monitoring, as only a limited number of sequences can be analyzed by conventional cloning, and sequencing of the TCR-CDR3 region might lead to biased results or deliver incomplete information.

Hence, methods found in the state of the art are dissatisfactory when applied to diagnostic objectives where sensitivity higher than a few percent is required. One prominent example is the monitoring of T cell lymphoma patients, where quantification of tumour cells is important for monitoring both treatment efficiency and relapse occurrence. Especially for the latter objective, sensitivity in the percent range, as attained by current diagnostic methods, is of little clinical value. Sensitivity in the sub-per-mill range is desirable for relapse monitoring in order to commence relapse treatment as early as possible.

Conventional analysis of T cell heterogeneity has been limited to obtaining around one hundred sequences, making the method unsuitable for diagnostic objectives that require higher sensitivity or result accuracy than a few percent.

High-throughput or "deep" sequencing methods have recently been developed, delivering hundreds of millions bases of sequence information in a single day. High throughput sequencing uses massively parallel sequencing reactions, reading out large numbers of relatively short sequences which are aligned by computing.

Different technology platforms may be chosen for this aim. Examples are so-called pyrosequencing and nanopore sequencing. The main difference between the three platforms most commonly employed consists of the method of in vitro clonal amplification, and of the sequencing procedure itself. The commercially available Roche 454 and Solid ABI platforms use emulsion PCR for isolation of individual DNA molecules from each other. These single molecules are coated by primer-beads in aqueous droplets within an oil phase. Amplification of clonal copies of the DNA molecule by PCR is followed by immobilization for later sequencing. In contrast, the Illumina Genome Analyzer platform uses so-called bridge PCR for in vitro clonal amplification, where fragments are amplified upon primers attached to a solid surface.

Sequencing by ligation is a further method available for high-throughput sequencing tasks.

The objective of the present invention is to provide a method for analysis of the T cell receptor repertoire in lymphocytes obtained from a patient. Such method should allow clonal characterization of said repertoire, should not be restricted to blood lymphocytes and should allow for a sensitivity of detection greater than available from methods of the state of the art.

This objective is attained by a method as specified in the independent claims.

According to one aspect of the invention, a method for analysis of a patient's T cell repertoire is provided, whereby
a. nucleic acid molecules encoding T cell receptor protein sequences are obtained from lymphocytes isolated from a patient;
b. a plurality of said nucleic acid molecules is sequenced, rendering sample TCR sequences;
c. the sample TCR sequences are aligned with a TCR sequence data base, and sample TCR sequences positively aligning to data base sequences are selected as selected sample TCR sequences,
d. the frequency distribution of at least some of the selected sample TCR sequences is determined.
Nucleic acid molecules may be DNA or RNA, for example messenger RNA or genomic DNA encoding T cell receptor protein sequences. In the context of this invention, T cell receptor protein sequences are sequences that confer antigen specificity on the T cell receptor, i.e. the hypervariable parts of the TCR protein chain that are generated during T cell maturation.
TCR nucleic acid sequences arise during T cell maturation by recombination of V, D and J gene segments for the generation of beta and delta chains, V and J segments in the case of alpha and gamma chains. Sequence variations arising from somatic hypermutation give rise to further complexity of possible sequences. Different sequences may be grouped according to their use of germline V-, D-and J-sequences as V-, D- or J-families.

Correspondingly, nucleic acid molecules encoding T cell receptor protein sequences are genomic or mRNA-sequences that encode these antigen-specificity-conferring TCR peptide sequences. The greatest variety is found in beta chain sequences, however alpha, gamma or delta chain sequences can be employed to practice the invention.

Aligning in the context of the present invention is the process of comparing each sample TCR sequence base by base with each TCR data base sequence, and quantifying the result in a comprehensible manner, preferably by a single parameter of similarity. Methods of alignment are known in the art; the FASTA and BLAST programme packages available publicly are examples. Sample TCR sequences that positively align to data base sequences show a degree of similarity to the latter that indicates that they have the same or highly similar antigen specificity.

The plurality of nucleic acid sequences that is sequenced in step b according to the above description of this aspect of the invention must be a number sufficient to give rise to a result that provides statistically relevant information on frequency distributions of TCR sequences in the sub-percent range. A number that is sufficient to provide information in the sub-per-mill-range is preferred. Hence, the plurality of sequences may signify a number greater than one thousand, for example ten thousand, twenty-five thousand or fifty thousand. In experiments carried out by the inventors, it was found that in order to retrieve information about the major immunodominant T cell clones within a patient's response to CMV, obtaining one hundred thousand sample TCR sequences did not materially improve the information in comparison to a second sequencing reaction during the course of which only thirty thousand sample TCR sequences were obtained.

The number above which significant information is not added by increasing the number of sample TCR sequences however may vary depending on the specific medical context. If recurrence of T cell tumour entities or transplant rejection monitoring is the objective of the method, far higher sensitivities will be warranted than if obtaining a general picture of the T cell response is intended.

A surprising advantage of the method according to the invention is the fact that sample TCR sequence distributions obtained by methods of the state of the art are qualitatively different from TCR sequence distributions obtained by the present invention. The methods of the state of the art make use of recombinant insertion of PCR amplificates into plasmids (subcloning), and subsequent clone picking and individual sequence analysis. This method introduces bias on a number of levels, most prominently during the steps of the subcloning process, but also during bacteria colony growth and selection. It was found that when comparing sample TCR sequence distribution obtained by high throughput sequencing to a smaller sample TCR sequence set obtained by subcloning, the high-throughput method according to the present invention afforded a more diverse sequence set than the traditional method. The dominant clone in CMV-specific TCR molecules was present in between 13% and 22% of the sample TCR sequences obtained by a method according to the invention, in comparison to 38% to 60% of clones analysed by the traditional method. These figures relate to the same original T cell pool, obtained from an identical patient.

Sample TCR sequences may be in the form of nucleic acid (NA) sequences or amino acid (AA) sequences. The sequencing reaction will render NA sequences, which can then be translated to AA sequences using the known triplet code. Comparison is performed advantageously on the level of AA sequences, as the biologically meaningful comparison to determine TCR specificity is performed on the AA sequence level.

Many applications of the method according to this aspect of the invention will use a TCR sequence database that is constituted of sequences derived from antigen-specific TCRs. In this case, the selected sample TCR sequences will be antigen-specific TCR sequences. Such TCR sequence data base characteristic of antigen-specific T cells will be heterogeneous and will comprise a great variety of different TCR sequences, according to the heterogeneity of the T cell response mounted against the antigen.

Other applications however, such as T cell lymphoma monitoring, or monitoring of adoptively transferred T cells, will use a TCR sequence data base comprised of a few or one single TCR sequence characteristic of the T cell clone, the frequency of which is to be monitored.

Hence, according to one preferred embodiment of the present invention, the TCR sequence data base comprises TCR sequences specific for either a T cell derived tumour having been obtained from said patient previously, or for an adoptively transferred T cell previously transferred into said patient.

One important advantage of this aspect of the present invention is the significant improvement in sensitivity when applied to monitoring of T cell derived tumours. Both monitoring of treatment response and, even more importantly, relapse monitoring, require the detection of small changes in tumour cell frequency, or very small total numbers of tumour cells, to provide optimal information to the physician. Current methods that provide information in the small percent range do not satisfy this requirement, as a tumour cell frequency of a few percent is equivalent to detecting a full relapse, whereas detection of recidivistic tumour cells in the sub-per-mill range, as enabled by the present invention, facilitates the opportunity to restart aggressive treatment regimens early on during the relapse.

A TCR sequence database is preferred for comparison, which comprises a set of TCR sequences that are representative of TCR of antigen-specific T cells. Especially preferred is a TCR sequence data base that is generated by the steps of
a) stimulating lymphocytes isolated from a patient in vitro with one or several antigens;
b) selecting T cells responding to stimulation from said lymphocytes;
c) obtaining a plurality of nucleic acid sequences encoding T cell receptor proteins from the selected T cells.

The number corresponding to the plurality of nucleic acid molecules sequenced in step c) may vary according to type of immune response or T cell population studied. It must be sufficiently large to allow for a statistically relevant attribution of distribution frequencies to several dozen sequences in the sub-percent range, preferably in the sub-per-mill range. Hence, a plurality of sequences may signify one thousand, preferably a number significantly greater than one thousand, for example ten thousand, twenty-five thousand or fifty thousand.

Current methods of high throughput sequencing allow for the parallel sequencing of thousands of short sequences; such high numbers are certainly within the range of numbers intended. According to one preferred embodiment, at least one thousand, even more preferred more than ten thousand sample TCR sequences are obtained. The sample TCR sequences may be aligned to known TCR sequences in order to assign them to a known TCR V- or J-family, for example.

When applied in the context of immune response monitoring, the method according to the present invention provides significant advantages over methods known in the art, by facilitating an analysis of the clonal diversity of the immune response. This may be applied to monitor and manage immune responses that are desired, from a medical point of view, or responses that are to be avoided. One example of an application to monitor a desired immune response is vaccination. In vaccination, immunization regimes are preferred that elicit a deep and broad response, as defined by the number and variety, respectively, of T cells specific for the antigen used for vaccination. The breadth of T cell responses has proven predictive for the amount of protection achieved in field trials of HIV, and the principle is applicable to all other pathogens where T cell responses are required for mounting a protective immune response, such as plasmodium, mycobacterium or leishmania. By applying the method of the present invention to analysis of the T cell repertoire specific for the antigen, a clonal analysis of the diversity of the T cell response is facilitated. Moreover, by applying the present invention repeatedly over time, information about the temporal evolution of the T cell mediated immune response is facilitated at a molecular level.

Similarly, the method of the present invention greatly improves the monitoring of immune functions that are detrimental for the patient. A prominent example is the monitoring of allergen-specific responses during allergen immunotherapy (induction of tolerance). Another application is the monitoring of transplant recipients (graft rejection or acceptance/tolerance).

According to another aspect of the invention, a method to obtain antigen-specific T cells for adoptive T cell transfer is provided, whereby lymphocytes isolated from a patient are stimulated in vitro with one or several antigens, the antigens preferably being present at low concentration during stimulation. A low concentration of an antigen in this context is a concentration where only a fraction of T cells naturally capable of responding to the antigen respond. Preferred as "low concentration" is a concentration of antigen at which only 10-25% of the numbers of cells respond to the antigen concentration compared to a "normal" concentration at which the entire population of cells that may be activated do respond. If, by way of example, T cell stimulation is effected in cell culture at a "normal" concentration of 1 µg/µL, a "low" concentration would be 0.1 µg/µL or lower. If low concentrations of target protein peptides are employed for stimulation, only TC with highly affine T-cell receptors will react.

According to this aspect of the invention, T cells responding to stimulation by antigen(s) are selected, and a plurality of nucleic acid sequences are obtained from a sample or at least a subsample of the selected T cells. Subsequently, nucleic acid molecule sequences obtained in the previous step are selected according to their frequency among the plurality of sequences obtained in that step, giving rise to a set of preferred sequences. T cells comprising specific preferred T cell receptor molecule sequences are obtained by culturing individual clones originating from a sample of the cells selected according to their stimulation, analysis of the T cell receptor protein sequence of the clone, and selecting a clone comprising a desired T cell receptor protein sequence, or by transferring an nucleic acid sequence encoding the desired T cell protein sequence under control of an RNA polymerase promoter operable in a mammalian cell, into a T cell.

The person skilled in the art will be able to chose from a large number of technologies for transferring nucleic acids encoding desired protein sequences into T cells. These include viral gene transfer, for example by vectors derived from the adenovirus, adeno-associated virus, lentivirus or herpes virus families, and insertion of DNA expression constructs into cells.

According to one preferred embodiment of the invention, the lymphocytes are isolated from blood. In a more preferred embodiment, the lymphocytes are peripheral blood mononuclear cells (PBMC), especially T lymphocytes, or lymphocytes obtained by tissue biopsy.

According to another preferred embodiment of the invention, the isolated lymphocytes are regulatory T cells. In an alternative preferred embodiment, the isolated lymphocytes are effector T cells. One may also practice the invention using a combination of both effector and regulatory T cells.

According to a further preferred embodiment, lymphocytes are stimulated with a peptide library representing antigenic peptides, or whole protein antigen, e.g. overlapping peptide pools spanning the whole CMV-specific protein pp 65, or lysates of cytomegalovirus (CMV-), BK-virus (BKV-), or Epstein-Barr-Virus (EBV-) specific proteins.

According to yet another preferred embodiment, lymphocytes are selected according to their expression of stimulation markers. According to a more preferred embodiment, selection is achieved through sorting by fluorescence activated cell sorting (FACS) or magnetic cell sorting (MACS) according to the cells' expression of activation markers on the cell surface or within the cells. Examples for such activation markers are IFNg, IL-2, TNFa, CD69, and CD40L.

The nucleic acid molecules encoding T cell receptor protein sequences that are analyzed according to the invention, encode regions of the TCR that contribute to TCR specificity; they preferably encode a peptide chain contributing to the diversity of CDR3 variants that generate the vast number of possible TCR structures. According to another preferred embodiment, the nucleic acid molecules analyzed encoding T cell receptor protein sequences encode all or part of the CDR3 region of the TCR beta chain.

The examples of this specification show a method for the analysis of TCR-CDR3 repertoire in CMV-specific T cells. A marked clonal restriction was observed among the effector memory T cells generated from individual donors using a CMV pp 65 peptide library. Sequencing data on more than 100 000 different CMV-specific TCR sequences for each donor attest to oligoclonality in CMV-specific TCR repertoire, while a very high degree of inter-individual clonal diversity exists within the CMV T cell recognition spectrum.

One embodiment of the method according to the invention similarly allows for the tracing of the clonal composition of viral antigen-specific T cells in the clinical course of patients with or without virus re-activation, as well as in patients receiving adoptive virus-specific T cell therapy. A preferred embodiment is directed towards monitoring the CMV infection and re-activation. Analysis of CMV-specific cytotoxic T cell (CTL) responses is essential for the prevention and therapy of CMV re-activation.

Another embodiment of the present invention allows for the monitoring of naturally occurring or adoptively transferred antigen-specific T cells.

The utility of the information obtained by the present method resides in its qualitative and quantitative accuracy as regards the T cell receptor repertoire of the immune response within a specific individual, unprecedented by methods of the state of the art. The interpretation of this information will be left to the physician, as well as possible conclusions with view towards treatment of the patient from whom this information was obtained.

### Short description of the figures

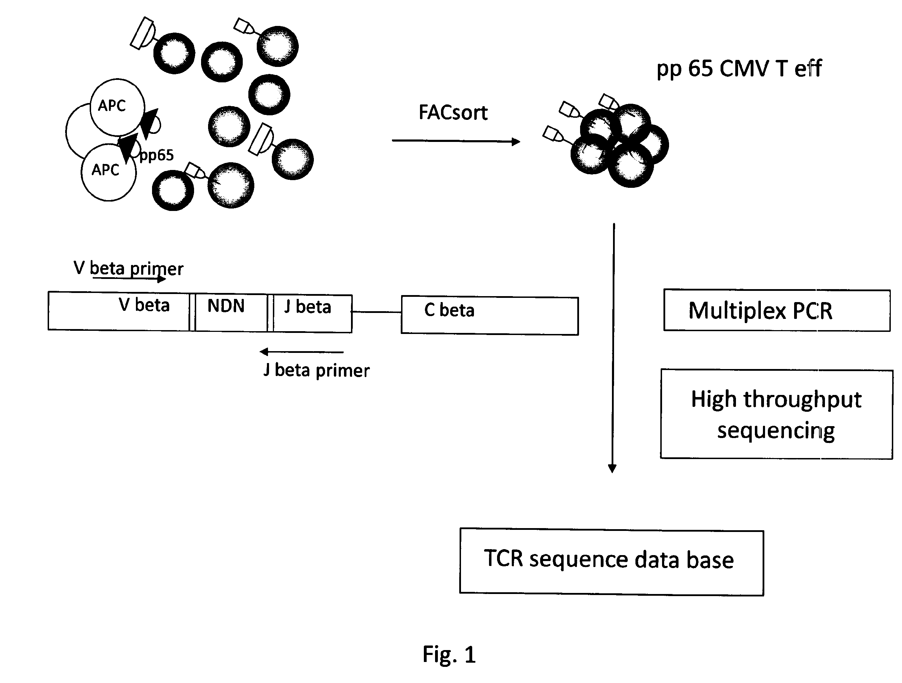

- Fig. 1: shows an example of a method to obtain the sequence data base for CMV pp65 specific TCR sequences.

### Examples

### Material and methods

Peripheral blood lymphocytes were isolated from a CMV-positive healthy donor. RNA was isolated as described previously (Babel et al, J. Am. Soc. Nephrol. 20; 344-352, 2009). The hypervariable regions of TCR Vβ chain was amplified by a 2-tube multiplex PCR with a primer mix that covers all Vβ TCR gene rearrangements on genomic DNA or cDNA samples as described previously (Babel et al, ibid.). RNA was isolated from separated cells by RNA Miniprep Kit (Stratagene, La Jolla, CA) according to the manufacturer's instructions. RNA was quantified by the Agilent 2100 Bioanalyzer System (Agilent Technologies, Santa Clara, CA) and used for cDNA synthesis. DNA was isolated using a commercially available DNA extraction kit (QIAamp Blood Kit, Qiagen, Hilden, Germany) according to the manufacturer's instructions. PCR products were separated on a 1.7 % agarose gel, excised, and purified using the Gel Extraction Kit (Eppendorf, Hamburg, Germany) following the manufacturer's instructions.

The purified product of multiplex PCR underwent nucleotide sequence analysis by high-throughput sequencing.

CDR3 junction sequences were obtained from 100.000 sequences. The frequency of the most prevalent sequences is given in table A below.

**Table A: CMV-specific sequences, 100.000 runs**

| SEQUENCE NAME | CDR3 JUNCTION (AA) | Length (nt) | # of reads | % |
|---|---|---|---|---|
| FXTBR9C02G4SL | C A S S P Q T G V G N Y G Y T F | 48 | 6648 | 22 |
| FXTBR9C02GWS | C A S S L L G Q G V N Q P Q H F | 51 | 1874 | 6 |
| FXTBR9C02GXBS | CS V S T G G W D Q P Q H F | 42 | 517 | 1.7 |
| FXTBR9C02G7SDM | C A S S F L T T T D T Q Y F | 42 | 508 | 1.69 |
| FXTBR9C02JEMV8 | C A S S L T S G A T Y N E Q F F | 48 | 348 | 1.16 |
| FXTBR9C02IW5QK | C A S S S G Q G Q V T Q Y F | 42 | 301 | 1.0 |

In a second experiment, CMV-specific sequences were obtained from a second individual in 30.000 runs (table B):

**Table B: CMV-specific sequences, 30.000 runs**

| SEQUENCE NAME | CDR3 JUNCTION (AA) | Length (nt) | # of reads | % |
|---|---|---|---|---|
| FXTBR9C02G4SL | C A S T W T G P D Q P Q H F | 42 | 12918 | 13 |
| FXTBR9C02I90TN | S V E V G V E G Y T F | 36 | 4952 | 5 |
| FXTBR9C02H7EFH | A S S R G A G N T E A F | 42 | 4524 | 4.5 |
| FXTBR9C02I0ONJ | C A S S A G L L Q E T Q Y F | 42 | 1570 | 1.57 |
| FXTBR9C02JDN7O | C A S S V Q G Y T E A F F | 39 | 1225 | 1.23 |
| FXTBR9C02I2LII | C A S S Q E V Q A P Y G Y T F | 45 | 803 | 0.8 |

## Claims

1. A method for analysis of a patient's T cell repertoire, whereby
a) nucleic acid molecules encoding T cell receptor protein sequences are obtained from lymphocytes isolated from a patient;
b) a plurality of said nucleic acid molecules obtained in step a) is sequenced, rendering sample TCR sequences;
c) the sample TCR sequences obtained in step b) are aligned with a TCR sequence data base, and sample TCR sequences positively aligning to data base sequences are selected as selected sample TCR sequences,
d) the frequency distribution of at least some of the selected sample TCR sequences obtained in step c) is determined.

2. The method of claim 1, whereby the TCR sequence data base is obtained by
a) stimulating lymphocytes isolated from a patient in vitro with one or several antigens;
b) selecting T cells responding to stimulation from said lymphocytes;
c) obtaining a plurality of nucleic acid sequences encoding T cell receptor proteins from at least some of the T cells selected in step b)).

3. The method according to claim 1, whereby the TCR sequence data base comprises TCR sequences specific for
a) a T cell derived tumour having been obtained from said patient previously, or
b) an adoptively transferred T cell previously injected into said patient.

4. A method to obtain antigen-specific T cells for adoptive T cell transfer, whereby
a) lymphocytes isolated from a patient are stimulated in vitro with one or several antigens;
b) T cells responding to stimulation are selected from said lymphocytes;
c) a plurality of nucleic acid molecule sequences encoding T cell receptor protein sequences is obtained from the T cells selected in step b);
d) nucleic acid molecule sequences obtained in step c) are selected according to their frequency among the plurality of sequences obtained in step c), rendering preferred sequences;
e) T cells comprising preferred sequences are obtained by
- culturing individual clones originating from a sample of the cells selected in step b), analysis of the nucleic acid molecule sequences encoding T cell receptor protein sequences of the clone, and selecting a clone comprising a preferred sequence, or
- transferring an operable nucleic acid sequence encoding a T cell protein sequence comprising a preferred sequence into a T cell.

5. A method according to claim 4, **characterized in that** the lymphocytes isolated from a patient are stimulated in vitro with one or several antigens at an antigen concentration equal or less than 25% of the antigen concentration employed to optimally stimulate lymphocytes.

6. A method according to at least one of the above claims, **characterized in that** the lymphocytes isolated from a patient are PBMC or lymphocytes obtained by tissue biopsy.

7. A method according to at least one of the above claims, **characterized in that** at least ten thousand sample TCR sequences are obtained.

8. A method according to at least one of the above claims, **characterized in that** the TCR sequence data base comprises TCR sequences of TCR molecules specific for antigens from any of the pathogens of the group including Epstein-Barr virus, Cytomegalovirus, plasmodium falciparum, mycobacterium tuberculosis, and/or Human Immunodeficiency Virus.

9. A method according to at least one of the above claims 1 to 3 and 6 to 8, **characterized in that** the TCR sequence data base comprises TCR sequences of TCR molecules specific for allergens or autoimmune antigens.

10. A method according to at least one of the above claims 2 to 9, **characterized in that** lymphocytes are stimulated by exposition to a peptide library representing antigenic peptides, or whole protein antigen.

11. Method according to at least one of the above claims 2 to 10, **characterized in that** lymphocytes are selected by fluorescence activated cell sorting (FACS) or magnetic cell sorting (MACS) according to the cells' expression of activation markers and/or cytokines on the cell surface or within the cells.

12. Method according to at least one of the above claims, **characterized in that** the nucleic acid molecules encoding T cell receptor protein sequences encode all or part of the CDR3 region of the TCR beta chain.

13. Cells obtained by a method according to at least one of claims 4 to 12.

14. Use of cells according to claim 12 as a pharmaceutical.

15. Use of cells according to claim 12 as a pharmaceutical in cancer therapy, treatment of transplant rejection, graft-versus-host-disease, viral infection, or vaccination.


SEQUENCE LISTING

<110>  Charité Universitätmedizin Berlin

<120>  High throughput analysis of T-cell receptor repertoires

<130>  ipa101ep

<160>  11

<170>  PatentIn version 3.5

<210>  1
<211>  16
<212>  PRT
<213>  Homo sapiens

<400>  1

Cys Ala Ser Ser Pro Gln Thr Gly Val Gly Asn Tyr Gly Tyr Thr Phe
1               5                   10                  15

<210>  2
<211>  16
<212>  PRT
<213>  Homo sapiens

<400>  2

Cys Ala Ser Ser Leu Leu Gly Gln Gly Val Asn Gln Pro Gln His Phe
1               5                   10                  15

<210>  3
<211>  14
<212>  PRT
<213>  Homo sapiens

<400>  3

Cys Ser Val Ser Thr Gly Gly Trp Asp Gln Pro Gln His Phe
1               5                   10

<210>  4
<211>  14
<212>  PRT
<213>  Homo sapiens

<400>  4

Cys Ala Ser Ser Phe Leu Thr Thr Thr Asp Thr Gln Tyr Phe
1               5                   10

<210>  5
<211>  16
<212>  PRT
<213>  Homo sapiens

<400>  5

Cys Ala Ser Ser Leu Thr Ser Gly Ala Thr Tyr Asn Glu Gln Phe Phe
1               5                   10                  15

<210>  6

<211>  14
<212>  PRT
<213>  Homo sapiens

<400>  6

Cys Ala Ser Ser Ser Gly Gln Gly Gln Val Thr Gln Tyr Phe
1               5                   10


<210>  7
<211>  12
<212>  PRT
<213>  Homo sapiens

<400>  7

Cys Ser Val Glu Val Gly Val Glu Gly Tyr Thr Phe
1               5                   10


<210>  8
<211>  14
<212>  PRT
<213>  Homo sapiens

<400>  8

Cys Ala Ser Ser Arg Gly Ala Gly Asn Thr Glu Ala Phe Phe
1               5                   10


<210>  9
<211>  14
<212>  PRT
<213>  Homo sapiens

<400>  9

Cys Ala Ser Ser Ala Gly Leu Leu Gln Glu Thr Gln Tyr Phe
1               5                   10


<210>  10
<211>  13
<212>  PRT
<213>  Homo sapiens

<400>  10

Cys Ala Ser Ser Val Gln Gly Tyr Thr Glu Ala Phe Phe
1               5                   10


<210>  11
<211>  15
<212>  PRT
<213>  Homo sapiens

<400>  11

Cys Ala Ser Ser Gln Glu Val Gln Ala Pro Tyr Gly Tyr Thr Phe
1               5                   10                  15